(19) Europäisches Patentamt
European Patent Office
Office européen des brevets

(11) Veröffentlichungsnummer: **0 081 462**
**B1**

# (12) EUROPÄISCHE PATENTSCHRIFT

(45) Veröffentlichungstag der Patentschrift: **18.01.89**

(21) Anmeldenummer: **82810518.9**

(22) Anmeldetag: **02.12.82**

(51) Int. Cl.⁴: **C 07 D 487/22**, C 09 B 47/24, C 09 B 47/26, A 01 N 43/90, C 11 D 3/00, D 06 M 16/00, C 02 F 1/32, C 02 F 1/50 // (C07D487/22, 259:00, 209:00, 209:00, 209:00, 209:00)

(54) **Wasserlösliche Zink- und Aluminiumphthalocyanine und deren Verwendung als Photoaktivatoren.**

(30) Priorität: **09.12.81 CH 7871/81**

(43) Veröffentlichungstag der Anmeldung: **15.06.83 Patentblatt 83/24**

(45) Bekanntmachung des Hinweises auf die Patenterteilung: **18.01.89 Patentblatt 89/3**

(84) Benannte Vertragsstaaten: **AT BE CH DE FR IT LI LU NL SE**

(56) Entgegenhaltungen:
**EP-A- 0 003 149**
**EP-A- 0 035 470**
**DE-A- 2 021 257**
**DE-A- 2 812 261**

(73) Patentinhaber: **CIBA-GEIGY AG, Klybeckstrasse 141, CH-4002 Basel (CH)**

(72) Erfinder: **Hölzle, Gerd, Dr., Spitzacker 9, CH-4410 Liestal (CH)**
Erfinder: **Miotto, Mirella, Dr., In den Klostermatten 17, CH-4052 Basel (CH)**
Erfinder: **Reinert, Gerhard, Dr., Weiherweg 1/7, CH-4123 Allschwil (CH)**
Erfinder: **Polony, Rudolf, Dr., Riehenstrasse 167, CH-4058 Basel (CH)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue wasserlösliche Zink- und Aluminiumphthalocyanine.

Aus der Literatur sind verschiedene Kupfer-, Nickel- und Kobaltphthalocyanine bekannt, die Sulfinsäure-, Cyanimid- oder Disulfimidgruppen enthalten und die als wasserlösliche Farbstoffe Verwendung finden. Siehe dazu unter anderem: deutsche Offenlegungsschriften 1 569 783, 1 569 729, 2 021 257, 1 794 298 und 1 419 840 sowie europäische veröffentlichte Patentanmeldung 24 677. Die in diesen Veröffentlichungen beschriebenen Farbstoffe sind jedoch nicht als Photoaktivatoren brauchbar und können daher nicht als wirksame Bleich- und antimikrobielle Mittel eingesetzt werden.

Ferner sind verschiedene wasserlösliche Phthalocyaninverbindungen, unter anderen auch Zink- und Aluminiumkomplexe, sowie Verfahren zum Bleichen von Textilien mit Hilfe dieser als Photoaktivatoren brauchbaren Verbindungen sowie Waschmittel, die diese Verbindungen enthalten, bekannt. Siehe dazu US-Patentschriften 3 927 967, 4 033 718, 4 166 718 und 4 094 806, deutsche Offenlegungsschriften 2 222 829 und 2 627 447 und veröffentlichte europäische Patentanmeldungen 35 470, 3149, 3371 und 3861. Ebenso ist ein Verfahren zur Bekämpfung von Mikroorganismen auf verschiedenen Substraten mit Hilfe der erwähnten wasserlöslichen Phthalocyaninverbindungen, z.B. wasserlöslichen Zink- und Aluminiumverbindungen, sowie ein diese Wirkstoffe enthaltendes Mittel aus der deutschen Offenlegungsschrift 2 812 261 bekannt.

Aufgabe der vorliegenden Erfindung war es nun, als Photoaktivatoren verwendbare wasserlösliche Phthalocyaninverbindungen zu finden, die besonders wirksam, wirtschaftlich und in vieler Hinsicht günstiger sind als die aus der Literatur bereits bekannten. Vor allem hinsichtlich ihrer Wirkung beim Bleichen von und Fleckenentfernen aus Textilien sowie bei ihrem Einsatz als antimikrobielle Wirksubstanzen sollten die zu schaffenden Verbindungen besonders gute und ausgiebige Effekte liefern. Ausserdem sollten sie in üblichen Wasch- und Reinigungsmitteln ohne Beeinträchtigung der Wirkung von anderen Komponenten einsetzbar sein. Ausserdem sollten die genannten Verbindungen leicht zugänglich und wirtschaftlich herstellbar sein.

Es wurde überraschenderweise gefunden, dass Zink- und Aluminiumphthalocyaninverbindungen, die mit Cyanimid-, Disulfimid- oder Sulfinsäuregruppen substituiert sind, den oben genannten Anforderungen entsprechen und daher die gestellte Aufgabe in hervorragender Weise lösen.

Die erfindungsgemässen neuen Phthalocyaninverbindungen entsprechen der allgemeinen Formel

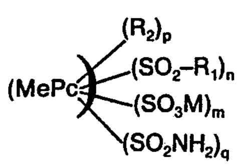

(1),

worin MePc für das Zink- oder Aluminiumphthalocyaninringsystem steht, M Wasserstoff oder das Äquivalent eines salzbildenden Kations, $R_1$ M oder eine Gruppe der Formel

$$-\underset{\displaystyle X}{\underset{|}{N}}-CN \text{ oder } -\underset{\displaystyle X}{\underset{|}{N}}-SO_2-R_3,$$

worin X für Wasserstoff oder Ammonium oder das Äquivalent eines ein-, zwei- oder dreiwertigen Metallions und $R_3$ unsubstituiertes oder durch Hydroxy, $C_1$–$C_8$-Alkoxy, Halogen, Cyano, Phenyl, Carb–$C_1$–$C_8$-alkoxy, Aminocarbonyl oder Di–$C_1$–$C_8$-alkylamino substituiertes $C_1$–$C_8$-Alkyl, unsubstituiertes oder durch $C_1$–$C_8$-Alkyl, $C_1$–$C_8$-Alkoxy, Nitro, Halo–$C_1$–$C_8$-alkyl, Halogen $C_1$–$C_8$-Alkoxycarbonyl, Cyano, $C_1$–$C_8$-Alkylsulfonyl, Carboxy und Sulfogruppen oder deren Salze, Ester oder Amide, Trifluormethyl oder Di-$C_1$–$C_8$-alkylamino substituiertes Phenyl, Naphthyl, einen unsubstituierten, substituierten und/oder anellierten 5- oder 6-gliedrigen, ein oder zwei Stickstoff-, Sauerstoff- oder Schwefelatome aufweisenden aromatischen heterocyclischen Ring, der wie die aromatische Gruppe $R_3$ substituiert sein kann, $R_2$ jeweils Halogen, Phenyl oder Cyano, n einen Wert von 1 bis 4, m und q jeweils einen Wert von 0 bis 3 und p einen Wert von 0 bis 4 bedeuten, wobei die Summe von n + m und n + q jeweils 1 bis 4 ist, q jedoch keine 0 ist, wenn $R_1$ eine Gruppe der Formel

$$-\underset{\displaystyle X}{\underset{|}{N}}-SO_2-R_3$$

und m = 0 sind.

Unter Naphthyl sind Naphthyl (1) oder Naphthyl (2) zu verstehen.

Die heterocyclischen aromatischen Ringe (Substituent $R_3$) enthalten gegebenenfalls einen ankondensierten Benzo- oder Naphthoring. Beispiele dafür sind der Benzofuran-, Thiophen-, Benzthiophen-, Benzoxazol-, Thiazol-, Benzthiazol-, Benzimidazol-, Pyridin-, Chinolinring. Im Fall von Heterocyclen mit ankondensiertem Benzo- oder Naphthoring können diese selbstverständlich auch über den ankondensierten Ring an die $SO_2$-Gruppe gebunden sein.

Die Phenyl, Naphthyl sowie die heteroaromatischen Ringe (inklusive solche mit ankondensierten Benzoringen) können durch verschiedene der oben genannten Reste substituiert sein, beispielsweise durch 1 bis 3 Substituenten, insbesondere einen Substituenten.

Halogen ist insbesondere Fluor, Chlor oder Brom, vorzugsweise Chlor.

Alkyl- und Alkoxygruppen haben als solche oder in Alkyl- oder Alkoxygruppen enthaltenden zusammengesetzten Gruppen 1 bis 8, insbesondere 1 bis 6, vorzugsweise 1 bis 4 C-Atome.

Bevorzugt bedeutet $R_2$ Chlor, Brom oder Jod, vor allem Chlor.

Die Sulfogruppen bzw. Sulfinsäuregruppen $-SO_2R_1$ im Phthalocyaninring können in freier Form oder als Salz vorliegen. Daher bedeutet M Wasserstoff oder ein salzbildendes Kation. Von den salzbildenden Kationen sind die Alkalimetall-, Erdalkalimetall-, Ammonium- und Aminsalzionen (= substituierte Ammoniumionen, die sich von primären, sekundären oder tertiären Aminen ableiten), bevorzugt. Insbesondere bedeutet M Wasserstoff, ein Alkalimetall-, Ammonium- oder Aminsalzion, vor allem Wasserstoff, Natrium, Kalium oder Ammonium.

Als Aminosalzionen (substituierte Ammoniumionen) sind z.B. solche der Formel

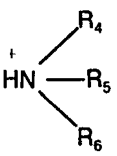

zu verstehen, worin $R_4$, $R_5$ und $R_6$ unabhängig voneinander Wasserstoff oder gegebenenfalls mit Halogen, Hydroxy, Phenyl oder Cyano substituiertes Alkyl (vorzugsweise mit 1–4 C-Atomen) bedeutet, wobei mindestens ein R-Substituent von Wasserstoff verschieden ist. Zwei R-Reste zusammen können auch die Ergänzung zu einem gesättigten 5- oder 6-gliedrigen Stickstoffheterocyclus bilden, der gegebenenfalls noch zusätzlich ein Sauerstoff- oder Stickstoffatom als Ringglied enthält. Beispiele für solche Heterocyclen sind: Piperidin, Piperazin, Morpholin, Pyrrolidin, Imidazolidin usw.

Die Gruppen $-NX-CN$ und $-NX-SO_2-R_3$ als Substituenten $R_1$ können ebenfalls als solche oder in Salzform vorliegen. Daher bedeutet X neben Wasserstoff Ammonium oder das Äquivalent eines ein-, zwei- oder dreiwertigen Metallions. Bevorzugt ist X Wasserstoff, Ammonium oder ein Alkalimetallion, z.B. ein Natrium- oder Kaliumion.

Die Indices n, m, p und q (sofern m bzw. q und/oder p nicht ohnehin null bedeuten), können beliebige Zahlen im angegebenen Bereich darstellen. Wie in der Phthalocyaninchemie üblich, bestehen die einzelnen Produkte häufig aus Mischungen, da bei der Herstellung (Sulfonierung, Sulfochlorierung, Halogenierung usw.) oft keine einheitlichen Produkte entstehen. Die Indices zeigen daher den «Substitutionsgrad» an, der selbstverständlich nicht ganzzahlig sein muss.

Die Zahl der im Molekül benötigten wasserlöslichmachenden Gruppen, d.h. Gruppen der Formel $-SO_2R_1$ + Sulfogruppen, hängt auch von der Zahl der vorhandenen Substituenten $R_2$ ab. Es müssen in jedem Fall so viele der erstgenannten Gruppen vorhanden sein, dass eine ausreichende Wasserlöslichkeit gewährleistet ist. Es kann bereits eine Mindestlöslichkeit von 0,01 g/l ausreichend sein, im allgemeinen ist eine solche von 0,1 bis 20 g/l zweckmässig.

Wie aus der Phthalocyaninchemie bekannt, ist im Aluminiumphthalocyaninringsystem die dritte Valenz des Aluminiums durch einen zusätzlichen Liganden, z.B ein Anion, abgesättigt. Dieses Anion kann mit dem Anion der Aluminiumverbindung identisch sein, die zur Herstellung des Komplexes benutzt wurde. Beispiele für solche Anionen sind Halogenid-, Sulfat-, Nitrat-, Acetat- oder Hydroxylionen.

Die Substituenten $-SO_2R_1$, $R_2$ und $-SO_3M$ bzw. $-SO_2NH_2$ sind, wie aus der Phthalocyaninchemie bekannt, vorzugsweise in den 3- und/oder 4-Stellungen der carbocyclischen aromatischen Ringe des Phthalocyaninringsystems gebunden. Es können jedoch auch andere Stellungen, zumindest teilweise, mit diesen Substituenten besetzt sein.

Besonders zu erwähnen sind Phthalocyaninverbindungen der Formel (1), worin M Wasserstoff, ein Alkalimetall-, Ammonium- oder Aminsalzion, $R_2$ ein Halogenatom oder Cyano, X Wasserstoff oder ein Ammonium- oder Alkalimetallatom, $R_3$ unsubstituiertes oder mit Hydroxy, Halogen, Alkoxy, Cyano, Phenyl, Carbalkoxy, Dialkylamino oder Aminocarbonyl substituiertes Alkyl, unsubstituiertes oder mit Alkyl, Alkoxy, Halogen, Nitro, Alkylsulfonyl, Cyano, Sulfo oder Carboxy und deren Derivate, oder Dialkylamino substituiertes Phenyl oder Naphthyl oder einen 5- oder 6-gliedrigen aromatischen heterocyclischen Ring, der 1–2 Heteroatome aus der Gruppe Stickstoff, Sauerstoff und Schwefel enthalten kann und der gegebenenfalls substituiert sein oder/und einen ankondensierten Benzo- oder Naphthorest tragen kann, bedeuten, und die Summe n + m bzw. n + q 2 bis 4 beträgt.

Von besonderem Interesse sind jene Verbindungen der Formel (1), worin $R_2$ ein Halogenatom und $R_3$ $C_1$–$C_4$-Alkyl, $C_1$–$C_4$-Halogenalkyl, $C_2$–$C_4$-Alkoxyalkyl, $C_1$–$C_4$-Hydroxyalkyl, $C_2$–$C_5$-Cyanoalkyl, Di-$C_1$–$C_4$-alkylamino-$C_1$–$C_4$-alkyl oder Benzyl, Phenyl oder mit 1–3 Substituenten aus der Gruppe Halogen, $C_1$–$C_4$-Alkyl, $C_1$–$C_4$-Alkoxy, Cyano, Nitro, Di-$C_1$–$C_4$-alkylamino und Sulfo und Carboxy und deren Salze substituiertes Phenyl bedeuten, insbesondere jene der Formel

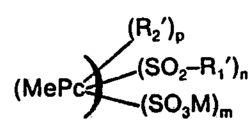

(2),

worin $R_2'$ Chlor, Brom oder Jod, insbesondere Chlor, M' Wasserstoff, Natrium, Kalium oder Ammonium und $R_1'$ Wasserstoff, M' oder eine Gruppe der Formel

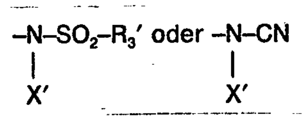

bedeuten, worin X' für Wasserstoff, Natrium, Kalium oder Ammonium und $R_3'$ für $C_1$–$C_4$-Alkyl, Phenyl, $C_1$–$C_4$-Alkylphenyl, Chlorphenyl oder Methoxyphenyl stehen und MePc, n, m und p wie in Formel (1) definiert sind.

Bevorzugt sind unter den Verbindungen der Formel (2) jene, in denen $R_2'$ Chlor, M' Wasser-

stoff, Natrium oder Kalium, X′ Wasserstoff, Natrium oder Kalium, $R_3'$ $C_1$–$C_4$-Alkyl, Phenyl oder $C_1$–$C_4$-Alkylphenyl, p eine Zahl von 0,5 bis 1,5, n eine Zahl von 2 bis 4 und m null bedeuten, insbesondere jene, in denen $R_2'$ Chlor, M′ Wasserstoff oder Natrium, $R_1'$ eine Gruppe

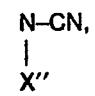

worin X″ für Wasserstoff oder Natrium steht, p eine Zahl von 0,5 bis 1,5, n eine Zahl von 3 bis 4 und m null bedeuten.

Von den Verbindungen der Formel (1) sind auch jene hervorzuheben, worin q 0 ist.

Als besonders vorteilhaft zu erwähnen sind jene Phthalocyaninverbindungen der Formeln (1) und (2), worin $R_1$ bzw. $R_1'$ für Wasserstoff oder eine Gruppe der Formel

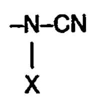 bzw. 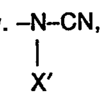

vorzugsweise für die Gruppe der Formel

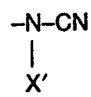

steht.

Die erfindungsgemässen Phthalocyaninverbindungen können nach an sich bekannten Verfahren erhalten werden. Zweckmässig stellt man Verbindungen der Formel (1) in der Weise her, dass man ein Moläquivalent eines Phthalocyaninsulfohalogenids der Formel

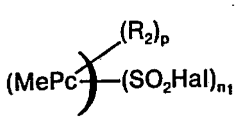 (3),

worin MePc, $R_2$ und p wie in Formel (1) definiert sind, Hal ein Halogenatom, insbesondere ein Chloratom bedeutet und $n_1$ eine beliebige Zahl zwischen 1 und 4 ist,

a) mit $n_1'$ Moläquivalenten Cyanamid oder einem Salz davon oder

b) mit $n_1'$ Moläquivalenten Ammoniak und das erhaltene Sulfonamid mit $n_1$ bzw. $n_1''$ Moläquivalenten eines Halogenids der Formel

$$Hal–SO_2–R_3,$$

worin Hal wie oben und $R_3$ wie in Formel (1) definiert ist, oder

c) mit $n_1'$ Moläquivalenten einer Verbindung der Formel $H_2N–Y$, worin Y für Amino, gegebenenfalls substituiertes Alkylamino oder Dialkylamino, Cyclohexylamino oder N-Acylamino steht, vorzugsweise mit Hydrazin,

umsetzt, wobei $n_1'$ bzw. $n_1''$ jeweils eine Zahl $\leqslant n_1$ ist, wobei im Fall b) $n_1$ Moläquivalent Ammoniak verwendet werden, wenn die Umsetzung mit $n_1''$ Moläquivalent $Hal–SO_2–R_3$ erfolgt, und gegebenenfalls gleichzeitig oder nachträglich in gemäss a) bis c) erhaltenen Produkten, die noch Sulfohalogenidgruppen enthalten, letztere zu Sulfogruppen hydrolysiert und gegebenenfalls erhaltene Verbindungen in die entsprechenden Salze überführt oder aus erhaltenen Salzen die entsprechenden sauren Formen freisetzt.

Zur Herstellung von Phthalocyaninverbindungen der Formel (1), worin $R_1$ eine Gruppe der Formel $–NXSO_2–R_3$ ist, insbesondere von solchen der Formel

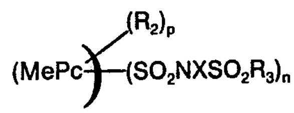 (4)

geht man zweckmässig von Verbindungen der Formel (3), insbesondere von solchen der Formel

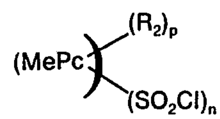 (5)

aus und setzt 1 Moläquivalent der letzteren mit $n_1'$ bzw. n Moläquivalent Ammoniak und das erhaltene Sulfonamid mit $n_1'$, n bzw. $n_1''$ Moläquivalent eines Sulfohalogenids der Formel $Hal–SO_2–R_3$, insbesondere eines Sulfochlorids der Formel $Cl–SO_2–R_3$, um. Erhaltene Verbindungen mit nicht umgesetzten Sulfochloridgruppen werden anschliessend hydrolysiert und gegebenenfalls Verbindungen mit M bzw. X = H in Salze übergeführt. Wenn die Verbindung der Formel (5) zuerst mit n Moläquivalent Ammoniak und anschliessend mit $n_1''$ Moläquivalent des Sulfohalogenids umgesetzt wird, erhält man Verbindungen der Formel (1) mit $m = 0$ und $q \neq 0$

Die Umsetzung erfolgt bevorzugt bei Temperaturen zwischen etwa 0 und 100 °C, insbesondere 30–50 °C, in wässrigem oder organisch-wässrigem Medium und pH-Werten oberhalb von 7, bevorzugt bei pH-Werten von etwa 10, wobei die entsprechenden Salze entstehen. Der pH-Wert wird dabei bevorzugt durch Zugabe alkalisch reagierender Verbindungen wie Alkalihydroxide oder -carbonate eingestellt.

Die Salze der erhaltenen Verbindungen, insbesondere die Alkalisalze, sind im allgemeinen in Wasser gut löslich und werden aus der Reaktionsmischung durch Aussalzen isoliert. Durch Ansäuern mit starken Mineralsäuren lassen sich die schwer löslichen freien Säuren isolieren. Geeignete gegebenenfalls mitzuverwendende organische Lösungsmittel sind Alkohole wie Methanol, Äthanol oder Propanol, Ketone wie Aceton, Amide wie Formamid, Dimethylformamid; Dimethylsulfoxid, Tetrahydrofuran, Chlorkohlenwasserstoffe wie Chloroform, ferner aromatische Kohlenwasserstoffe wie Benzol oder Toluol.

Alternativ können die Verbindungen der Formel (1) mit $R_1 = -NX-SO_2-R_3$ auch durch Umsetzung von Phthalocyaninsulfohalogeniden (insbesondere -chlorien) der Formel (3) mit Sulfonamiden der Formel $R_3-SO_2-NH_2$ erhalten werden. Bevorzugte Reaktionsparameter entsprechen jenen für das vorstehend beschriebene Verfahren angeführten.

Zur Herstellung von Phthalocyaninverbindungen der Formel (1), worin $R_1$ eine Gruppe $-NX-CN$ ist, insbesondere von solchen der Formel

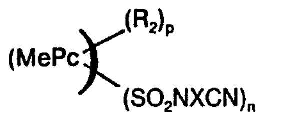

(6)

geht man zweckmässig von Verbindungen der Formel (3), insbesondere von solchen der Formel (5) aus und setzt ein Moläquivalent einer solchen Verbindung mit $n_1'$ bzw. n Moläquivalent Cyanamid bzw. einem Salz davon um. Erhaltene Verbindungen mit nicht umgesetzten Sulfochloridgruppen werden anschliessend hydrolysiert und gegebenenfalls Verbindungen mit M bzw. X = H in Salze übergeführt.

Die Umsetzung erfolgt vorzugsweise in wässrigem Medium oder in einem inerten organischen Lösungsmittel, das vorzugsweise mit Wasser mischbar ist, oder in einem Gemisch aus Wasser und organischem Lösungsmittel, bei einem pH-Wert zwischen 4 und 14, insbesondere zwischen 9 und 14, und bei einer Temperatur zwischen $-10$ und 110 °C, insbesondere zwischen 0 und 40 °C. Als mit Wasser mischbare Lösungsmittel kommen beispielsweise ein niederer Alkohol, wie Methanol oder Äthanol, N-Methylpyrrolidon, Dimethylformamid, Dimethylsulfoxid oder Mischungen davon in Betracht.

Cyanamid kann auch in Form seiner Salze eingesetzt werden, beispielsweise als Mono- oder Dinatriumcyanamid oder Calciumcyanamid.

Als Basen (säurebindende Mittel), die bei der Kondensationsreaktion eines Phthalocyaninsulfochlorids mit Cyanamid oder dessen Salzen bzw. mit einem Amid $R_3-SO_2-NH_2$ oder eines Phthalocyaninsulfonamids (aus Halogenid + Ammoniak) mit einem Halogenid $R_3-SO_2-Hal$ zur Herstellung von Verbindungen der allgemeinen Formel (1) eingesetzt werden können, sind bevorzugt Alkali- und Erdalkalihydroxide und basisch reagierende Salze der Alkali- und Erdalkalimetalle mit anorganischen oder organischen Säuren oder tertiäre organische Basen. Unter Alkalimetallen werden vorzugsweise Natirum und Kalium verstanden, unter Erdalkalimetallen vorzugsweise Calcium. Basisch reagierende Salze sind bevorzugt die Alkalimetallsalze der Kohlensäure, der Phosphorsäure und der Essigsäure, wie insbesondere Natrium- und Kaliumacetat, Natrium- und Kaliumbicarbonat, Natrium- und Kaliumcarbonat, Natriumdihydrogenphosphat, Dinatrium-hydrogenphosphat, Trinatriumphosphat oder deren entsprechende Kaliumsalze. Tertiäre organische Basen sind beispielsweise Pyridin, Triäthanolamin oder Dimethylanilin.

Zur Herstellung von Phthalocyaninverbindungen der Formel (1) mit $R_1$ = H geht man ebenfalls von den Sulfohalogeniden der Formel (3) aus, die man mit einem Hydrazinderivat der Formel $H_2N-Y$, worin Y für Amino, gegebenenfalls substituiertes Alkylamino oder Dialkylamino, Cyclohexylamino oder N-Acylamino steht, umsetzt, vorzugsweise mit Hydrazin selbst. Diese Umsetzung findet ebenfalls vorzugsweise in wässrigem Medium statt. Beispielsweise kann die Umsetzung bei pH-Werten zwischen 5 und 14 durchgeführt werden (siehe nachfolgende Beispiele 5, 5.1 und 5.2).

Alternativ können die Sulfohalogenid (insbesondere -chlorid)gruppen der als Ausgangsverbindungen eingesetzten Phthalocyaninsulfohalogenide (insbesondere -chloride) auch nach folgendem Schema:

$$-SO_2Cl + Na_2SO_3 + 2\,NaOH \rightarrow -SO_2Na + NaCl + Na_2SO_4 + H_2O$$

zu den entsprechenden Sulfinsäuregruppen umgesetzt werden. Diese Methode kann ebenfalls für die Herstellung der erfindungsgemässen Phthalocyaninverbindungen der Formel (1), worin $R_1$ = H bzw. M, eingesetzt werden. Diese Methode kann nach der in Houben-Weyl, Bd 9, S. 299ff. (1955) angegebenen Vorschrift ausgeführt werden.

Wie bereits aus der allgemeinen Verfahrensbeschreibung ersichtlich, können Verbindungen der Formel (1), in denen m nicht gleich null ist, erhalten werden, indem man vor, während oder nach der Umwandlung der $-SO_2Cl$-Gruppen zu $-SO_2R_1$-Gruppen einen Teil der Sulfochloridgruppen der Ausgangsverbindungen durch Hydrolyse in Sulfonsäuregruppen überführt. So kann die Hauptreaktion beispielsweise so durchgeführt werden, dass gleichzeitig mit der Umsetzung ein Teil der Sulfonsäurechloridgruppen hydrolysiert wird, oder es kann so verfahren werden, dass zunächst mit einer zur vollständigen Umsetzung aller Sulfonsäurechloridgruppen nicht ausreichenden Menge umgesetzt wird und dass anschliessend die restlichen Sulfonsäurechloridgruppen durch einen besonderen Reaktionsschritt in saurem oder alkalischem Milieu, so bei einem pH-Wert zwischen 1 und 4 oder zwischen 8 und 12, gegebenenfalls in der Wärme, so bei Temperaturen zwischen 20 und 60 °C, hydrolysiert werden.

Die Isolierung der nach den vorstehend beschriebenen Verfahren erhaltenen Phthalocyaninverbindungen erfolgt in üblicher Weise durch Aussalzen, beispielsweise mit Natrium-, Kalium- oder Ammoniumchlorid, und/oder durch Ansäuern mit einer Mineralsäure oder durch Eindampfen von deren neutralen oder schwach sauren wässrigen Lösungen, vorzugsweise bei mässig erhöhter Temperatur und vermindertem Druck. Die erfindungsgemässen Phthalocyaninverbindungen werden dementsprechend in Form ihrer Natrium-, Kalium- oder Ammoniumsalze oder in Säureform oder in einem Gemisch dieser Formen erhalten.

Selbstverständlich können, wenn Verbindungen der Formel (1) mit m $\neq$ 0 gewünscht werden, auch Ausgangsverbindungen verwendet werden, die

neben Sulfochloridgruppen (und gegebenenfalls $R_2$-Gruppen) bereits Sulfogruppen enthalten.

Substituenten $R_2$, insbesondere Halogenatome, können auch nachträglich durch Halogenierung in Verbindung der Formel (1) mit $p = 0$ eingeführt werden.

Die Herstellung der als Ausgangsprodukte eingesetzten Sulfohalogenide (Sulfochloride) der Formel (3) bzw. (5) kann nach bekannten Methoden durch Reaktion der Zink- und Aluminiumphthalocyanine, die gegebenenfalls auch bereits Substituenten $R_2$ enthalten, mit einer Halogensulfonsäure, insbesondere mit Chlorsulfonsäure erfolgen. Derartige Reaktionen sind in der Literatur betreffend Phthalocyanine ausführlich beschrieben.

Der Aufbau des Phthalocyaninringgerüstes aus Derivaten der Phthalsäure, wobei chlorierte Phthalocyanine entstehen, ist in Ullmann's Encyclopädie der technischen Chemie, 4. Aufl., Band 18, Seite 507ff und bei F.H. Moser, A.L. Thomas, «Phthalocyanine» (1963), Seite 104ff beschrieben. Halogenierte bzw. sonstige inerte Substituenten tragende Phthalocyanine können durch Mischkondensation von unsubstituierten bzw. entsprechend substituierten Phthalsäuren bzw. Phthalsäurederivaten nach üblichen, in der Phthalocyaninchemie bekannten Verfahren erhalten werden. Sofern der Aufbau des Phthalocyaninringsystems aus Phthalsäureanhydrid oder Phthalodinitril in Gegenwart von Chloriden, z.B. $AlCl_3$ oder $ZnCl_2$, durchgeführt wird, entstehen beretis chlorierte Phthalocyanine, besonders mit einem Chlorgehalt von 0,5–1,5 Mol Chlor pro Mol Aluminiumphthalocyanin.

Die erfindungsgemässen Phthalocyaninverbindungen können, wie eingangs erwähnt, als Photoaktivatoren verwendet werden, insbesondere zum Bekämpfen von Mikroorganismen, vor allem aber zum Bleichen von bzw. zum Fleckenentfernen aus Textilien. In der Literatur wird an Stelle des Ausdrucks «Photoaktivator» oft auch den Ausdruck «Photosensibilisator» verwendet. Letzterer Ausdruck könnte daher auch für die in dieser Anmeldung verwendete Bezeichnung «Photoaktivator» stehen.

In den folgenden Beispielen stellen Teile- und Prozentangaben immer Gewichtsteile und Gwichtsprozent dar, sofern nichts anderes angegeben ist. In allen Beispielen bedeutet AlPc das Aluminiumphthalocyaninringsystem und ZnPc das Zinkphthalocyaninringsystem. Die zur Charakterisierung der Phthalocyaninverbindungen verwendeten $\lambda_{max}$-Werte aus dem Absorptionsspektrum wurden in wässriger Lösung bei pH 7 bestimmt.

Beispiel 1:

60 Teile Monochlor-aluminiumphthalocyanin werden unter gutem Rühren in 260 Vol.-Teile Chlorsulfonsäure eingetragen. Durch Aussenkühlung wird die Temperatur bei 20 bis 25 °C gehalten. Das Reaktionsgemisch wird zuerst eine halbe Stunde bei Raumtemperatur gerührt, dann wird die Temperatur innerhalb einer Stunde auf 110 bis 115 °C erhöht. Nach einer halben Stunde wird die Reaktionstemperatur innerhalb einer Stunde auf 130 bis 135 °C gesteigert und vier Stunden eingehalten. Hieraut wird das Reaktionsgemisch auf 70 bis 75 °C abgekühlt und innerhalb 45 Minuten mit 125 Vol.-Teilen Thionylchlorid versetzt. Man rührt eine weitere Stunde bei 85 bis 90 °C, lässt dann die Reaktionsmasse auf Raumtemperatur abkühlen und trägt sie anschliessend auf eine Eis/Wasser-Mischung aus. Die kalte Sulfochloridsuspension wird abgenutscht und mit Eiswasser säurefrei gewaschen.

Die so erhaltene Sulfochloridpaste wird in 1200 Teilen Eis/Wasser suspendiert, mit 21 Teilen Cyanamid versetzt und mit Natronlauge auf pH 10 gehalten. Das Reaktionsgemisch wird bei Raumtemperatur so lange gerührt, bis der pH-Wert ohne weitere Laugenzugabe konstant bleibt. Die entstandene Lösung wird zur Trockne eingedampft. Man erhält 148 Teile eines blauen Pulvers.

50 Teile des erhaltenen Pulvers werden in 500 Teilen Wasser gelöst, mit konz. Salzsäure auf pH 2 gestellt und zur Trockne eingedampft. Der Rückstand wird fein pulverisiert und dann in 500 Volumenteilen 1N Salzsäure verrührt.

Die Suspension wird filtriert und der Rückstand mit 250 Volumenteilen 1N Salzsäure gewaschen. Das Filtergut wird in 500 Teilen Wasser angerührt, mit Natronlauge auf pH 7 gestellt und anschliessend eingedampft. Man erhält 32 Teile eines blauen Pulvers.

Die so erhaltene Verbindung entspricht der Formel

$$\underset{\displaystyle |\atop \displaystyle Cl}{AlPc}-\left[SO_2-\overset{\ominus}{N}-CN\quad Na^{\oplus}\right]_{ca.\,3} \qquad (101)$$

($\lambda_{max} = 675$ nm).

Beispiel 1.1:

Verfährt man nach der Vorschrift von Beispiel 1, ersetzt jedoch Monochlor-aluminiumphthalocyanin durch eine äquivalente Menge an unsubstituiertem Aluminiumphthalocyanin, so erhält man 29 Teile der Verbindung der Formel

$$AlPc-(SO_2\overset{\ominus}{N}-CN\quad Na^{\oplus})\quad ca.\ 4 \qquad (102)$$

in Form eines blauen Pulvers mit einem $\lambda_{max}$ von 675 nm.

Beispiel 1.2:

Verfährt man nach der Vorschrift von Beispiel 1, setzt jedoch die Sulfochloridpaste nur mit 4,5 Teilen bzw. 9 Teilen Cyanamid um, so erhält man die Verbindung der Formel

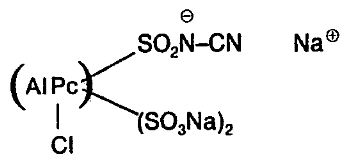

(103)

mit einem $\lambda_{max}$ von 677 nm bzw. der Formel

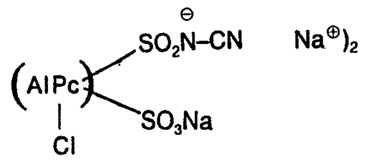

(104)

mit einem $\lambda_{max}$ von 677 nm.

Beispiel 2:

60 Teile Zinkphthalocyanin werden unter gutem Rühren in 260 Vol.-Teile Chlorsulfonsäure eingetragen. Durch Aussenkühlung wird dabei die Temperatur bei 20 bis 25 °C gehalten. Das Reaktionsgemisch wird zuerst eine halbe Stunde bei Raumtemperatur gerührt; dann erhöht man die Temperatur innerhalb einer Stunde auf 110 bis 115 °C. Nach einer halben Stunde wird die Reaktionstemperatur innerhalb einer Stunde auf 130 bis 135 °C gesteigert und 4 Stunden eingehalten. Hierauf wird das Reaktionsgemisch auf 70 bis 75 °C abgekühlt und innerhalb 45 Minuten mit 125 Vol.-Teilen Thionylchlorid versetzt. Man rührt eine weitere Stunde bei 85 bis 90 °C, lässt dann die Reaktionsmasse auf Raumtemperatur abkühlen und trägt sie anschliessend auf eine Eis/Wasser-Mischung aus. Die kalte Sulfochloridsuspension wird abgenutscht und mit Eiswasser säurefrei gewaschen.

Die so erhaltene feuchte Sulfochloridpaste wird in 1200 Teilen Eis/Wasser suspendiert, mit 21 Teilen Cyanamid versetzt und mit Natronlauge auf pH 10 gehalten. Das Reaktionsgemisch wird bei Raumtemperatur so lange gerührt, bis der pH-Wert ohne weitere Laugenzugabe konstant bleibt.

Die entstandene Lösung wird klärfiltriert, mit 3 Teilen Zinkchlorid versetzt, mit Natronlauge auf pH 12 gestellt und 2 Stunden bei 40 bis 45 °C gerührt. Dann wird die Lösung mit konz. Salzsäure auf pH 1 gestellt und zur Trockne eingedampft. Der Rückstand wird fein pulverisiert und dann in 500 Teilen Wasser 1 Stunde lang verrührt. Die Suspension wird filtriert und das Filtergut mit wenig Wasser gewaschen. Der Rückstand wird in 1000 Teilen Wasser verrührt, mit Natronlauge auf pH 7 gestellt und die entstandene Lösung zur Trockne eingedampft. Man erhält 55 Teile eines blauen Pulvers.

Die so erhaltene Verbindung entspricht der Formel

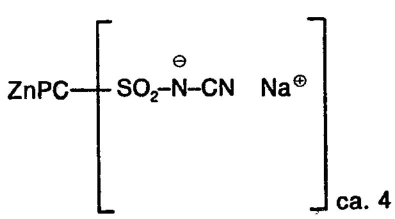

(201)

($\lambda_{max}$ = 671 nm)

Beispiel 2.1:

Verfährt man nach der Vorschrift von Beispiel 2, setzt jedoch die Sulfochloridpaste nur mit 4,5, 9 bzw. 13,5 Teilen Cyanamid um, so erhält man die Verbindungen der Formeln

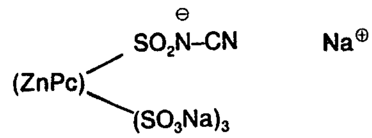

, (202)

($\lambda_{max}$ = 670 nm),

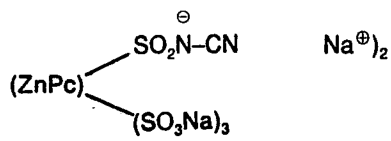

(203)

($\lambda_{max}$ = 670 nm) bzw.

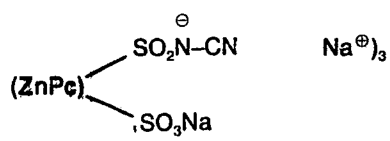

(204)

($\lambda_{max}$ = 670 nm).

Beispiel 3:

Die nach Beispiel 1, Absatz 1 erhaltene Sulfochloridpaste wird in 1200 Teilen Eis/Wasser suspendiert und im Rührkolben mit 100 Volumenteilen konz. Ammoniak versetzt. Nach zweistündigem Rühren wird die Reaktionstemperatur auf 55 bis 60 °C erhöht und während 12 Stunden gerührt. Die entstandene Lösung wird zur Trockne eingedampft und der Rückstand pulverisiert. Das Pulver wird in 600 Volumenteilen 1N Salzsäure 1 Stunde lang verrührt, dann abfiltriert, mit 300 Volumenteilen 1N Salzsäure gewaschen und anschliessend getrocknet. Man erhält 86 Teile eines grünblauen Pulvers, das etwa der Formel

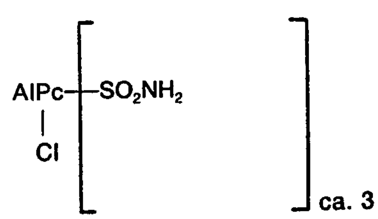

(300)

entspricht.

21,5 Teile des erhaltenen Pulver werden in 300 Teilen Wasser verrührt, mit Natronlauge auf pH 10

gestellt und bei Raumtemperatur tropfenweise mit 14 Teilen Methansulfochlorid versetzt. Dann wird das Reaktionsgemisch 2 Stunden bei 20 bis 25 °C und anschliessend 2 Stunden bei 60 °C gerührt. Durch Zutropfen von Natronlauge wird dabei der pH-Wert bei 10 gehalten. Die entstandene Lösung wird zur Trockne eingedampft. Der Rückstand wird pulverisiert, dann in 300 Volumenteilen 2N Salzsäure verrührt, abfiltriert und mit 100 Volumenteilen 2N Salzsäure gewaschen. Das Nutschgut wird in 300 Teilen Wasser wieder angerührt, mit Natronlauge auf pH 7 gestellt und zur Trockne eingedampft. Man erhält so 24 Teile der Verbindung der Formel:

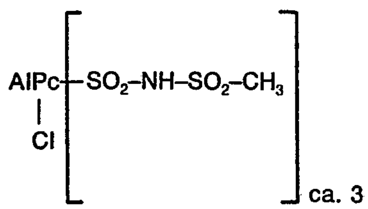

(301)

($\lambda_{max}$ = 673/637 nm).

Beispiel 3.1:

Verfährt man nach der Vorschrift von Beispiel 3, setzt jedoch die Verbindung der Formel (300) mit nur 3 bzw. 6 Teilen Methansulfochlorid um, so erhält man die Verbindung der Formel

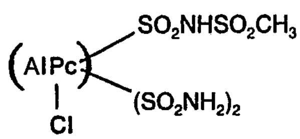

(302)

($\lambda_{max}$ = 637/679 nm) bzw. der Formel

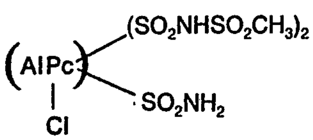

(303)

($\lambda_{max}$ = 637/675 nm)

Beispiel 3.2:

Verfährt man nach der Vorschrift von Beispiel 3, setzt aber statt Methansulfochlorid 15 Teile, 6,4 Teile bzw. 3,2 Teile Toluol-4-sulfochlorid, gelöst in 60, 25 bzw. 12 Teilen Aceton, ein, so erhält man die Verbindungen der Formeln

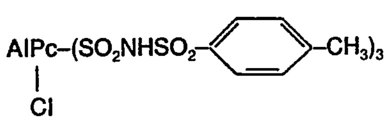

(304)

($\lambda_{max}$ = 677 nm),

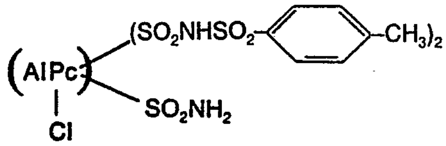

(305)

($\lambda_{max}$ = 677 nm), bzw.

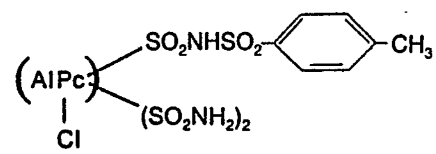

(306)

($\lambda_{max}$ = 676 nm).

Beispiel 3.3:

Die nach Beispiel 1, Absatz 1 erhaltene Sulfochloridpaste suspendiert man in 1200 Teilen Eis/Wasser und gibt 26 Teile Toluol-4-sulfamid zu. Der pH-Wert des Reaktionsgemisches wird mit Natronlauge bei 10–11 gehalten. Die Reaktionstemperatur lässt man auf 20–25 °C ansteigen, erhitzt dann auf 50–55 °C, bis keine Lauge mehr verbraucht wird. Die Reaktionslösung wird auf 25 °C abgekühlt, mit Salzsäure auf pH 7,5 eingestellt und dann klärfiltriert. Das Filtrat wird zur Trockne eingedampft. Es werden 151 Teile des Natriumsalzes der Verbindung der Formel

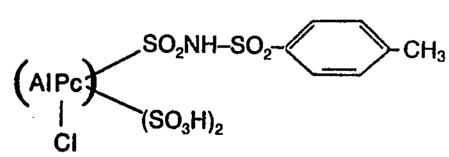

(307)

in Form eines blauen Pulvers mit einem $\lambda_{max}$ von 676 nm erhalten.

Beispiel 3.4:

Verfährt man nach der Vorschrift von Beispiel 3.3, ersetzt jedoch die nach Beispiel 1, Absatz 1 erhaltene Sulfochloridpaste durch jene nach Beispiel 2, Absatz 1 erhaltene, so fallen 101 Teile des Natriumsalzes der Verbindung der Formel

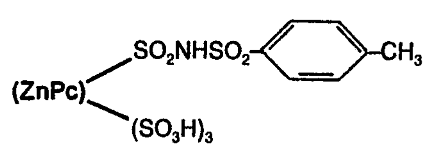

(308)

in Form eines blauen Pulvers mit einem $\lambda_{max}$ von 629 nm an.

Beispiel 4:

Die nach Beispiel 2, Absatz 1 erhaltene Sulfochloridpaste wird in 1200 Eis/Wasser suspendiert und im Rührkolben mit 100 Volumenteilen konz. Ammoniak versetzt. Nach zweistündigem Rühren wird die Reaktionstemperatur auf 55 bis 60 °C erhöht und während 12 Stunden gerührt. Die entstandene Lösung wird zur Trockne eingedampft und der Rückstand pulverisiert. Das Pulver wird in 600 Volumenteilen 1N Salzsäure 1 Stunde lang verrührt, dann abfiltriert, mit 300 Volumenteilen 1N Salzsäure gewaschen und anschliessend getrocknet. Man erhält ein grünblaues Pulver, das etwa der Formel

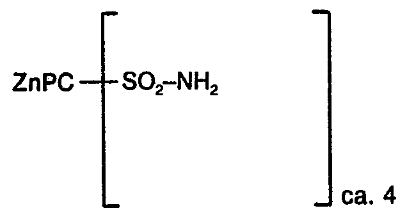

(400)

entspricht.

17,5 Teile des erhaltenen Zinkphthalocyaninsulfonamids werden in 300 Teilen Wasser angerührt, mit Natronlauge auf pH 11 gestellt und bei 80 bis 90 °C portionenweise mit 22 Teilen Toluol-4-sulfochlorid versetzt. Durch Zutropfen von Natronlauge wird der pH-Wert bei 10 bis 11 gehalten. Nach zweistündigem Rühren lässt man die Lösung auf 40 bis 45 °C abkühlen, stellt mit Natronlauge auf pH 12, setzt 1 Teil Zinkchlorid zu und rührt weitere 2 Stunden bei 40 bis 45 °C. Dann wird die Lösung mit Salzsäure auf pH 0,5 gestellt. Das ausgefallene Produkt wird abfiltriert und mit verdünnter Salzsäure gewaschen. Das Nutschgut wird in 300 Teilen Wasser suspendiert, mit Natronlauge auf pH 7,5 gestellt und die entstandene Lösung zur Trockne eingedampft. Man erhält so 20 Teile der Verbindung der Formel:

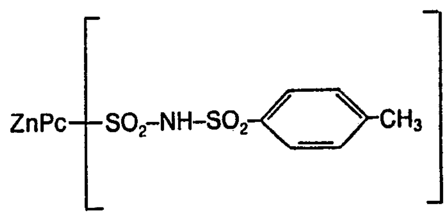

(401)

($\lambda_{max}$ = 628 nm).

Beispiel 4.1:

Verfährt man nach der Vorschrift von Beispiel 4, setzt jedoch die Verbindung der Formel (400) mit nur 11 Teilen Toluol-4-sulfochlorid um, so erhält man die Verbindung der Formel

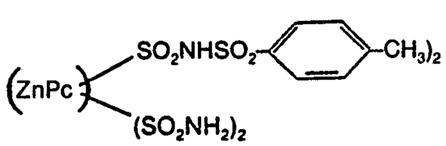

(402)

($\lambda_{max}$ = 629 nm).

Beispiel 4.2:

Verfährt man nach der Vorschrift von Beispiel 4, setzt jedoch die Verbindung der Formel (400) mit 8 Teilen Methansulfochlorid um, so erhält man die Verbindung der Formel

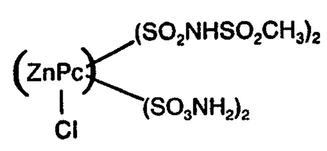

(403)

($\lambda_{max}$ = 627 nm).

Beispiel 5:

Die nach Beispiel 1, Absatz 1 erhaltene Sulfochloridpaste wird in 1200 Teilen Eis/Wasser suspendiert und mit Natronlauge auf pH 6,5 gestellt. Dann werden 20 Teile Hydrazinhydrat zugegeben. Man lässt die Reaktionstemperatur auf 20 bis 25 °C steigen und hält den pH-Wert durch Zutropfen von Natronlauge zwischen 6,5 und 7,5. Dabei ist eine starke Stickstoffentwicklung zu beobachten. Nach 2 Stunden gibt man bis zur Erreichung von pH 10 Natronlauge zu und rührt 10 Stunden lang. Die Lösung wird dann mit konz. Salzsäure auf pH 1 gestellt. Das ausgefallene Produkt wird abfiltriert, in 1000 Teilen Wasser suspendiert, mit Natronlauge auf pH 7,5 gestellt und zur Trockne eingedampft. Man erhält so 132 Teile des Natriumsalzes der Verbindung der Formel

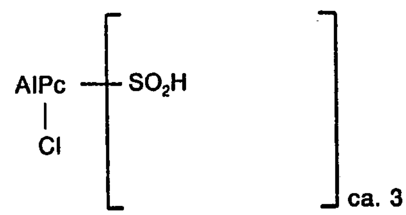

(501)

($\lambda_{max}$ = 682 nm).

Beispiel 5.1:

Verfährt man wie in Beispiel 5 beschrieben, setzt jedoch die Sulfochloridpaste nur mit 10 bzw. 5 Teilen Hydrazinhydrat um, so erhält man das Natriumsalz der Verbindung der Formel

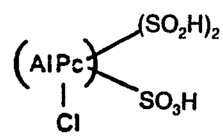

(502)

($\lambda_{max}$ = 682 nm) bzw. der Formel

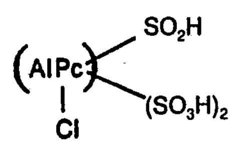

(503)

($\lambda_{max}$ = 679 nm)

Beispiel 5.2:

Verwendet man an Stelle der nach Beispiel 1, Absatz 1 erhaltenen Sulfochloridpaste jene nach Beispiel 2, Absatz 1 erhaltene (Zinkphthalocyaninsulfochlorid) und setzt letztere wie in Beispiel 5 beschrieben mit Hydrazinhydrat um, wobei 20, 15, 10 bzw. 5 Teile Hydrazinhydrat eingesetzt werden, so kommt man zu den Natriumsalzen der Verbindungen der Formeln

$ZnPC-(SO_2H)_4$ (504)

($\lambda_{max}$ = 634 nm),

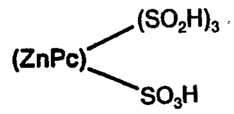

(505)

($\lambda_{max}$ = 633 nm),

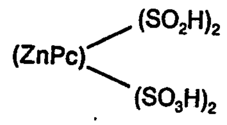

(506)

($\lambda_{max}$ 633 nm) bzw.

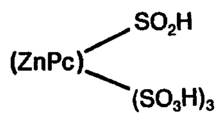

(507)

($\lambda_{max}$ = 632 nm).

Beispiel 6:

Das in den Beispielen 1, 3 und 5 als Ausgangsprodukt verwendete Monochlor-aluminiumphthalocyanin kann nach folgenden Herstellungsvorschriften erhalten werden:

a) In einem Autoklaven werden 128 g Phthalsäuredinitril, 40 g $AlCl_3$ und 650 g 1,2-Dichlorbenzol vorgelegt. Nach Spülung mit Stickstoff wird die Reaktionsmasse 26 Stunden auf etwa 170 °C erhitzt. Nach Abkühlen und Entlüften wird die Suspension unter Rühren in 400 ml Wasser, enthaltend 100 g Trinatriumphosphat, gegossen. Danach wird auf dem Rotationsverdampfer zur Trockne eingedampft, das Rohprodukt mit 750 ml Wasser angerührt, mit 60 g 50%iger NaOH versetzt, auf 75 °C erhitzt und 2 Stunden bei dieser Temperatur gehalten. Schliesslich wird das Rohprodukt abfiltriert, in 500 ml Wasser, enthaltend 80 g 32%ige HCl, verrührt (2 Stunden bei 90–95 °C), heiss filtriert und gewaschen. Man erhält so Aluminiumphthalocyanin, das pro Mol etwa 1 Mol Chlor gebunden enthält (Monochloraluminiumphthalocyanin).

b) In einem Rührkolben werden 118 Teile Harnstoff, 20 Teile 4-Chlorphthalsäure, 44,4 Teile Phthalsäureanhydrid, 27 Teile Xylolsulfonsäure (Isomerengemisch), 1 Teil Ammoniummolybdat, 15 Teile Aluminiumchlorid und 200 Vol. Teile Trichlorbenzol (Isomerengemisch) gut verrührt, innerhalb 3 Stunden auf 195 bis 205 °C erwärmt und bei dieser Temperatur 16 Stunden gerührt. Nach dem Erkalten werden 500 Vol. Teile Isopropanol zugegeben, kurz verrührt und dann wird die Suspension abgenutscht. Der Rückstand wird mit 500 Vol. Teilen Isopropanol gewaschen, in 800 Vol. Teilen verdünnter Natronlauge aufgenommen, 2 Stunden bei 80 bis 90 °C gerührt, dann abgenutscht und mit warmem Wasser gewaschen. Dann wird der gleiche Prozess in verdünnter Salzsäure durchgeführt, das erhaltene Pigment mit warmem Wasser säurefrei gewaschen und getrocknet. Es werden ca. 50 Teile Monochloraluminiumphthalocyanin in Form eines blauen Pulvers erhalten.

**Patentansprüche für die Vertragsstaaten: BE, CH, DE, FR, IT, LU, NL, SE, LI**

1. Phthalocyaninverbindungen der Formel

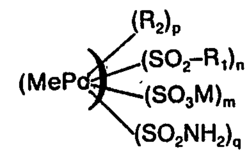

worin MePc das Zink- oder Aluminiumphthalocyaninringsystem, M Wasserstoff oder das Äquivalent eines salzbildenden Kations und $R_1$ M oder eine Gruppe der Formeln

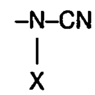 oder 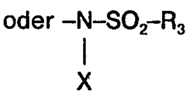

bedeuten, in denen X Wasserstoff, Ammonium oder das Äquivalent eines ein-, zwei- oder dreiwertigen Metallions, $R_3$ unsubstituiertes oder durch Hydroxy, $C_1$–$C_8$-Alkoxy, Halogen, Cyano, Phenyl, Carb–$C_1$–$C_8$-alkoxy, Aminocarbonyl oder Di–$C_1$–$C_8$-alkylamino substituiertes $C_1$–$C_8$-Alkyl, unsubstituiertes oder durch $C_1$–$C_8$-Alkyl, $C_1$–$C_8$-Alkoxy, Nitro, Halo–$C_1$–$C_8$-alkyl, Halogen, $C_1$–$C_8$-Alkoxycarbonyl, Cyano, $C_1$–$C_8$-Alkylsulfonyl, Carboxy und Sulfogruppen oder deren Salze, Ester oder Amide, Trifluormethyl oder Di–$C_1$–$C_8$-alkylamino substituiertes Phenyl, Naphthyl, einen unsubstituierten, substituierten und/oder anellierten 5- oder 6-gliedrigen, ein oder zwei Stickstoff-, Sauerstoff- oder Schwefelatome aufweisenden aromatischen heterocyclischen Ring, der wie die aromatische Gruppe $R_3$ substituiert sein kann, $R_2$ jeweils Halogen, Phenyl oder Cyano, n einen Wert von 1 bis 4, m und q jeweils einen Wert von 0 bis 3 und p einen Wert von 0 bis 4 bedeuten, wobei die Summe von n + m und n + q jeweils 1 bis 4 ist, q jedoch keine 0 ist, wenn $R_1$ eine Gruppe der Formel

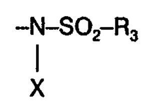

und m = 0 sind.

2. Phthalocyaninverbindungen gemäss Anspruch 1, worin q = 0 ist.

3. Phthalocyaninverbindungen gemäss Anspruch 1, worin M Wasserstoff, ein Alkalimetall-, Ammonium- oder Aminsalzion, $R_2$ Halogen oder Cyano und X Wasserstoff, Ammonium oder Alkalimetall bedeuten.

4. Phthalocyaninverbindungen gemäss Anspruch 3, worin $R_2$ Halogen und $R_3$ $C_{1-4}$-Alkyl, $C_{1-4}$-Haloalkyl, $C_{2-6}$-Alkoxyalkyl, $C_{1-4}$-Hydroxyalkyl, $C_{2-5}$-Cyanoalkyl, Di($C_{1-4}$-alkyl)-amino-$C_{1-4}$-alkyl,

Benzyl, Phenyl, durch 1 bis 3 Halogen, $C_{1-4}$-Alkyl, $C_{1-4}$-Alkoxy, Cyano, Nitro, Di-($C_{1-4}$-alkyl)amino oder Sulfo substituiertes Phenyl oder eine Carboxygruppe und deren Salze bedeuten.

5. Phthalocyaninverbindungen gemäss Anspruch 4, gekennzeichnet durch die Formel

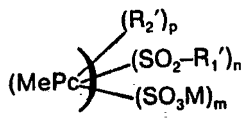

worin $R_2'$ Chlor, Brom oder Jod, M' Wasserstoff, Natrium, Kalium oder Ammonium und $R_1'$ M' oder eine Gruppe der Formeln

$-N-SO_2-R_3'$ oder $-N-CN$
| |
X' X'

bedeuten, in denen X' Wasserstoff, Natrium, Kalium oder Ammonium, $R_3'$ $C_{1-4}$-Alkyl, Phenyl, oder $C_{1-4}$-Alkylphenyl, Chlorphenyl oder Methoxyphenyl bedeuten und MePc, n, m und p im Anspruch 1 definiert sind.

6. Phthalocyaninverbindungen gemäss Anspruch 2, worin $R_1$ Wasserstoff oder eine Gruppe der Formel

$-N-CN$
|
X

bedeutet.

7. Phthalocyaninverbindungen gemäss Anspruch 5, worin $R_2'$ Chlor bedeutet.

8. Phthalocyaninverbindungen gemäss Anspruch 5, worin $R_1'$ eine Gruppe der Formel

$-N-CN$
|
X'

bedeutet.

**Patentansprüche für den Vertragsstaat AT**

1. Verfahren zur Herstellung von Phthalocyaninverbindungen der Formel

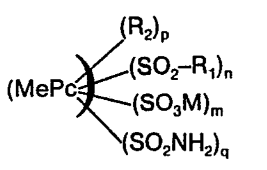 (1),

worin MePc das Zink- oder Aluminiumphthalocyaninringsystem, M Wasserstoff oder das Äquivalent eines salzbildenden Kations und $R_1$ M oder eine Gruppe der Formeln

$-N-CN$ oder $-N-SO_2-R_3$,
| |
X X

bedeuten, in denen X Wasserstoff, Ammonium oder das Äquivalent eines ein-, zwei- oder dreiwertigen Metallions, $R_3$ unsubstituiertes oder durch Hydroxy, $C_1$–$C_8$-Alkoxy, Halogen, Cyano, Phenyl, Carb-$C_1$–$C_8$-alkoxy, Aminocarbonyl oder Di-$C_1$–$C_8$-alkylamino substituiertes $C_1$–$C_8$-Alkyl, unsubstituiertes oder durch $C_1$–$C_8$-Alkyl, $C_1$–$C_8$-Alkoxy, Nitro, Halo-$C_1$–$C_8$-alkyl, Halogen, $C_1$–$C_8$-Alkoxycarbonyl, Cyano, $C_1$–$C_8$-Alkylsulfonyl, Carboxy und Sulfogruppen oder deren Salze, Ester oder Amide, Trifluormethyl oder Di-$C_1$–$C_8$-alkylamino substituiertes Phenyl, Naphthyl, einen unsubstituierten, substituierten und/oder anellierten 5- oder 6-gliedrigen, ein oder zwei Stickstoff-, Sauerstoff- oder Schwefelatome aufweisenden aromatischen heterocyclischen Ring, der wie die aromatische Gruppe $R_3$ substituiert sein kann, $R_2$ jeweils Halogen, Phenyl oder Cyano, n einen Wert von 1 bis 4, m und q jeweils einen Wert von 0 bis 3 und p einen Wert von 0 bis 4 bedeuten, wobei die Summe von n + m und n + q jeweils 1 bis 4 ist, q jedoch keine 0 ist, wenn $R_1$ eine Gruppe der Formel

$-N-SO_2-R_3$
|
X

und m = 0 sind, dadurch gekennzeichnet, dass man ein Moläquivalent eines Phthalocyaninsulfohalogenids der Formel

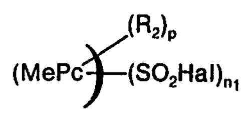 (3),

worin MePc, $R_2$ und p wie oben definiert sind, Hal ein Halogenatom, insbesondere ein Chloratom bedeutet und $n_1$ eine beliebige Zahl zwischen 1 und 4 ist,

a) mit $n_1'$ Moläquivalent Cyanamid oder einem Salz davon oder

b) mit $n_1'$ Moläquivalent Ammoniak und das erhaltene Sulfonamid mit $n_1$ bzw. $n_1''$ Moläquivalent eines Halogenids der Formel

$$Hal-SO_2-R_3,$$

worin Hal und $R_3$ wie oben definiert ist, oder

c) mit $n_1'$ Moläquivalent einer Verbindung der Formel $H_2N-Y$, worin Y für Amino, gegebenenfalls substituiertes Alkylamino oder Dialkylamino, Cyclohexylamino oder N-Acylamino steht, vorzugsweise mit Hydrazin,

umsetzt,

wobei $n_1'$ bzw. $n_1''$ jeweils eine Zahl $\leqslant n_1$ ist, wobei im Fall b) $n_1$ Moläquivalent Ammoniak verwendet werden, wenn die Umsetzung mit $n_1''$ Moläquivalenten $Hal-SO_2-R_3$ erfolgt, und gegebenenfalls gleichzeitig oder nachträglich in gemäss a) bis c) erhaltenen Produkten, die noch Sulfohalogenidgruppen enthalten, letztere zu Sulfogruppen hydrolysiert und gegebenenfalls erhaltene Verbindungen in die entsprechenden Salze überführt

oder aus erhaltenen Salzen die entsprechenden sauren Formen freisetzt.

2. Verfahren nach Anspruch 1 zur Herstellung von Phthalocyaninverbindungen der Formel (1), worin q für 0 steht, dadurch gekennzeichnet, dass man im Fall b) die Umsetzung des Sulfonamids mit $n_1$ Moläquivalent des Halogenids der Formel Hal—$SO_2$–$R_3$ durchführt.

3. Verfahren nach Anspruch 1 oder 2 zur Herstellung von Phthalocyaninverbindungen der Formel (1), worin M Wasserstoff, ein Alkalimetall-, Ammonium- oder Aminsalzion, $R_2$ ein Halogenatom oder Cyano, X Wasserstoff, Ammonium oder ein Alkalimetallatom bedeuten, dadurch gekennzeichnet, dass man eine Verbindung der Formel (3), worin $R_2$ jeweils wie vorstehend und die übrigen Symbole wie in Anspruch 1 definiert sind und $n_1$ eine beliebige Zahl zwischen 2 und 4 ist, gemäss Reaktion (a), b) oder c) umsetzt, wobei $R_3$ im Halogenid Hal–$SO_2$–$R_3$ wie in Anspruch 1 definiert ist.

4. Verfahren nach Anspruch 3 worin $R_2$ ein Halogenatom und $R_3$ $C_1$–$C_4$-Alkyl, $C_1$–$C_4$-Halogenalkyl, $C_2$–$C_6$-Alkoxyalkyl, $C_1$–$C_4$-Hydroxyalkyl, $C_2$–$C_5$-Cyanoalkyl, Di-$C_1$–$C_4$-alkylamino-$C_1$–$C_4$-alkyl oder Benzyl, Phenyl oder mit 1–3 Substituenten aus der Gruppe Halogen, $C_1$–$C_4$-Alkyl, $C_1$–$C_4$-Alkoxy, Cyano, Nitro, Di-$C_1$–$C_4$-alkylamino und Sulfo und Carboxy bzw. deren Salze substituiertes Phenyl bedeuten, dadurch gekennzeichnet, dass man eine Verbindung der Formel (3), worin $R_2$ jeweils wie vorstehend und die übrigen Symbole wie in Anspruch 1 definiert sind und $n_1$ eine beliebige Zahl zwischen 2 und 4 ist, gemäss Reaktion a), b) oder c) umsetzt, wobei $R_3$ im Halogenid Hal–$SO_2$–$R_3$ wie vorstehend definiert ist.

5. Verfahren nach Anspruch 4 zur Herstellung von Phthalocyaninverbindungen der Formel

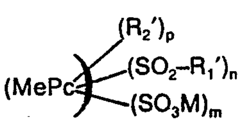 (2),

worin $R_2'$ Chlor, Brom oder Jod, insbesondere Chlor, M' Wasserstoff, Natrium, Kalium oder Ammonium und $R_1'$ Wasserstoff, M' oder eine Gruppe der Formel

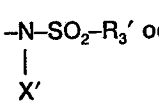 oder 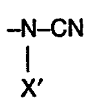

bedeuten, worin X' für Wasserstoff, Natrium Kalium oder Ammonium und $R_3'$ für $C_1$–$C_4$-Alkyl, Phenyl, $C_1$–$C_4$-Alkylphenyl, Chlorphenyl oder Methoxyphenyl stehen und MePc, n, m und p wie in Anspruch 3 definiert sind, vorzugsweise von solchen der Formel (2), worin $R_2'$ Chlor, M' Wasserstoff, Natrium oder Kalium, X' Wasserstoff, Natrium oder Kalium, $R_3'$ $C_1$–$C_4$-Alkyl, Phenyl oder $C_1$–$C_4$-Alkylphenyl, p eine Zahl von 0,5 bis 1,5, n eine Zahl von 2 bis 4 und m null bedeuten, dadurch gekennzeichnet, dass man ein Moläquivalent eines Phthalocyaninsulfohalogenids der Formel

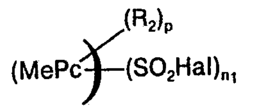 ,

worin $R_2'$ und p wie vorstehend definiert sind, Hal ein Halogenatom und $n_1$ eine beliebige Zahl von 1 und 4, vorzugsweise 2 bis 4, bedeuten,

a) mit $n_1'$, vorzugsweise $n_1$, Moläquivalent Cyanamid oder einem Salz davon oder

b) mit $n_1'$, vorzugsweise $n_1$, Moläquivalent Ammoniak und das erhaltene Sulfonamid mit $n_1$ Moläquivalent eines Halogenids der Formel Hal–$SO_2$–$R_3'$, worin $R_3'$ wie vorstehend definiert ist und Hal ein Halogen-, vorzugsweise ein Chloratom bedeutet, oder

c) mit $n_1'$, vorzugsweise $n_1$, Moläquivalent einer Verbindung der Formel $H_2N$–Y, worin Y für Amino, gegebenenfalls substituiertes Alkylamino oder Dialkylamino, Cyclohexylamino oder N-Acylamino steht, vorzugsweise mit Hydrazin, umsetzt,

wobei $n_1'$ eine Zahl $\leqslant n_1$ ist, und gegebenenfalls gleichzeitig oder nachträglich in gemäss a) bis c) erhaltenen Produkten, die noch Sulfohalogenidgruppen enthalten, letztere zu Sulfogruppen hydrolysiert.

6. Verfahren nach Anspruch 2 oder 5 zur Herstellung von Phthalocyaninverbindungen der Formel (1) bzw. (2), worin q 0 ist und $R_1$ bzw. $R_1'$ für Wasserstoff oder eine Gruppe der Formel

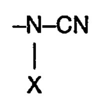 bzw. 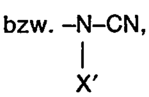 ,

vorzugsweise für eine Gruppe der Formel

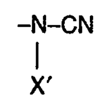

steht, dadurch gekennzeichnet, dass man ein Moläquivalent eines Sulfochlorids der Formel

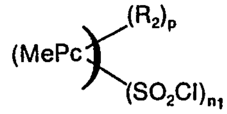 bzw. 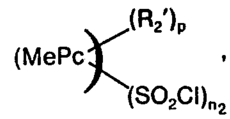 ,

worin $R_2$, $R_2'$, p und $n_1$ wie in den Ansprüchen 2 bzw. 5 definiert sind,

a) mit $n_1'$, vorzugsweise $n_1$, Moläquivalent Cyanamid oder einem Salz davon oder

b) mit $n_1'$, vorzugsweise $n_1$, Moläquivalent einer Verbindung der Formel $H_2N$–Y, worin Y wie in Anspruch 5 definiert ist, vorzugsweise mit Hydrazin, umsetzt, wobei $n_1'$ eine Zahl $\leqslant n_1$ ist und wobei die Umsetzung a) bevorzugt ist, und gegebenenfalls gleichzeitig oder nachträglich in gemäss a) oder b) erhaltenen Produkten, die noch

Sulfohalogenidgruppen enthalten, letztere zu Sulfogruppen hydrolysiert.

7. Verfahren zur Herstellung von Phthalocyaninverbindungen der Formel (1), worin $R_1$ eine Gruppe der Formel $-NXSO_2-R_3$ ist, dadurch gekennzeichnet, das man ein Moläquivalent eines Phthalocyaninsulfohalogenids der Formel (3) mit $n_1'$ Moläquivalent eines Sulfonamides der Formel $R_3-SO_3NH_2$, worin $R_3$ wie in Anspruch 1 definiert ist, umsetzt, wobei $n_1'$ eine Zahl $\leqslant n_1$ ist und gegebenenfalls gleichzeitig oder nachträglich in erhaltenen Produkten, die noch Sulfohalogenidgruppen enthalten, letztere zu Sulfogruppen hydrolysiert.

8. Verfahren nach Anspruch 1 zur Herstellung von Phthalocyaninverbindungen der Formel

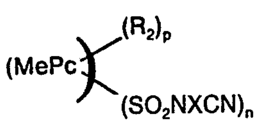

worin die allgemeinen Symbole die in Anspruch 1 angegebene Bedeutung haben, dadurch gekennzeichnet, das man ein Moläquivalent eines Sulfochlorids der Formel

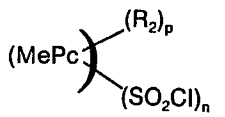

mit etwa n Moläquivalent Cyanamid umsetzt und gegebenenfalls die erhaltene Verbindung mit X = H in ein Salz überführt.

**Claims for the Contracting States: BE, CH, DE, FR, IT, LU, NL, SE, LI**

1. A phthalocyanine compound of the formula

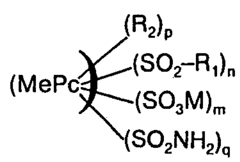

wherein MePc is the zinc or aluminium phthalocyanine ring system, M is hydrogen or the equivalent of a salt-forming cation and $R_1$ is M or a group of the formula

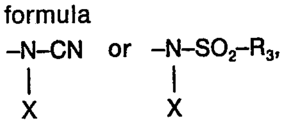

wherein X is hydrogen, ammonium or the equivalent of a monovalent, divalent or trivalent metal ion, $R_3$ is $C_1$–$C_8$alkyl which is unsubstituted or substituted by hydroxyl, $C_1$–$C_8$alkoxy, halogen, cyano, phenyl, carb-$C_1$–$C_8$alkoxy, aminocarbonyl or di-$C_1$–$C_8$alkylamino, or is phenyl which is unsubstituted or substituted by $C_1$–$C_8$alkyl, $C_1$–$C_8$alkoxy, nitro, halo-$C_1$–$C_8$alkyl, halogen, $C_1$–$C_8$alkoxycarbonyl, cyano, $C_1$–$C_8$alkylsulphonyl, carboxyl and sulpho groups or salts, esters or amides thereof, trifluoromethyl or di-$C_1$–$C_8$alkylamino, or is naphthyl, an unsubstituted, substituted and/or fused 5-membered or 6-membered aromatic heterocyclic ring which contains 1 or 2 nitrogen, oxygen or sulphur atoms and can be substituted in the same manner as the aromatic group $R_3$, $R_2$ is in each case halogen, phenyl or cyano, n has a value from 1 to 4, m and q each have a value from 0 to 3 and p has a value from 0 to 4, the sum of n + m and n + q in each case being 1 to 4, however, q is not 0, if $R_1$ is a group of the formula

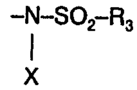

and m = 0.

2. A phthalocyanine compound according to claim 1, wherein q is 0.

3. A phthalocyanine compound according to claim 1, wherein M is hydrogen, an alkali metal ion, an ammonium ion or an amine salt ion, $R_2$ is halogen or cyano and X is hydrogen, ammonium or alkali metal.

4. A phthalocyanine compound according to claim 3, wherein $R_2$ is halogen and $R_3$ is $C_1$–$C_4$alkyl, $C_1$–$C_4$haloalkyl, $C_2$–$C_6$alkoxyalkyl, $C_1$–$C_4$hydroxyalkyl, $C_2$–$C_5$cyanoalkyl, di($C_1$–$C_4$alkyl)amino–$C_1$–$C_4$alkyl, benzyl, phenyl, phenyl substituted by 1 to 3 halogen, $C_1$–$C_4$alkyl, $C_1$–$C_4$alkoxy, cyano, nitro, di-($C_1$–$C_4$alkyl)amino or sulpho, or is a carboxyl group and the salts thereof.

5. A phthalocyanine compound according to claim 4 of the formula

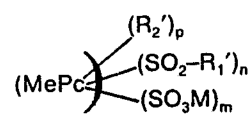

wherein $R_2'$ is chlorine, bromine or iodine, M' is hydrogen, sodium, potassium or ammonium and $R_1'$ is M' or a group of the formula

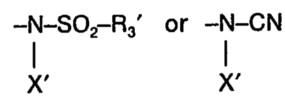

wherein X' is hydrogen, sodium, potassium or ammonium, $R_3'$ is $C_1$–$C_4$alkyl, phenyl, or $C_1$–$C_4$alkylphenyl, chlorophenyl or methoxyphenyl, and MePc, n, m and p are defined in claim 1.

6. A phthalocyanine compound according to claim 2, wherein $R_1$ is hydrogen or a group of the formula

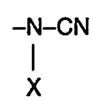

7. A phthalocyanine compound according to claim 5, wherein $R_2'$ is chlorine.

8. A phthalocyanine compound according to claim 5, wherein $R_1'$ is a group of the formula

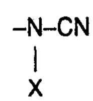

**Claims for the Contracting State AT**

1. A process for the preparation of a phthalocyanine compound of the formula

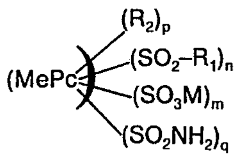

(1),

wherein MePc is the zinc or aluminium phthalocyanine ring sytem, M is hydrogen or the equivalent of a salt-forming cation and $R_1$ is M or a group of the formula

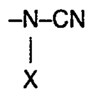 or 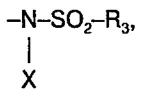,

wherein X is hydrogen, ammonium or the equivalent of a monovalent, divalent or trivalent metal ion, $R_3$ is $C_1$–$C_8$alkyl which is unsubstituted or substituted by hydroxyl, $C_1$–$C_8$alkoxy, halogen, cyano, phenyl, carb–$C_1$–$C_8$alkoxy, aminocarbonyl or di–$C_1$–$C_8$alkylamino, or is phenyl which is unsubstituted or substituted by $C_1$–$C_8$alkyl, $C_1$–$C_8$alkoxy, nitro, halo–$C_1$–$C_8$alkyl, halogen, $C_1$–$C_8$alkoxycarbonyl, cyano, $C_1$–$C_8$alkylsulphonyl, carboxyl and sulpho groups or salts, esters or amides thereof, trifluoromethyl or di–$C_1$–$C_8$alkylamino, or is naphthyl, an unsubstituted, substituted and/or fused 5-membered or 6-membered aromatic heterocyclic ring which contains 1 or 2 nitrogen, oxygen or sulphur atoms and can be substituted in the same manner as the aromatic group $R_3$, $R_2$ is in each case halogen, phenyl or cyano, n has a value from 1 to 4, m and q each have a value from 0 to 3 and p has a value from 0 to 4, the sum of n + m and n + q in each case being 1 to 4, however, q is not 0, if $R_1$ is a group of the formula

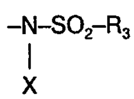

and m = 0.
which process comprises reacting one molar equivalent of a phthalocyanine sulphohalide of the formula

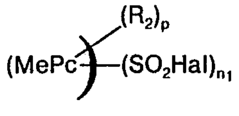

(3),

wherein MePc, $R_2$ and p are as defined above, Hal is a halogen atom, in particular a chlorine atom, and $n_1$ is any number between 1 and 4,

a) with $n_1'$ molar equivalents of cyanamide or a salt thereof, or

b) with $n_1'$ molar equivalents of ammonia, and reacting the resultant sulphonamide with $n_1$ or $n_1''$ molar equivalents of a halide of the formula

$$Hal–SO_2–R_3,$$

wherein Hal and $R_3$ are as defined above, or

c) with $n_1'$ molar equivalents of the compound of formula $N_2N$–Y, wherein Y is amino, unsubstituted or substituted alkyl amino or dialkylamino, cyclohexylamino or N-acylamino, preferably with hydrazine, where $n_1'$ and $n_1''$ are each a number $\leqslant n_1$, $n_1$ molar equivalents of ammonia being used in case b) if the reaction is carried out with $n_1''$ molar equivalents of $Hal–SO_2–R_3$ and, in a product obtained according to a) to c) which still contains sulphohalide groups, simultaneously or subsequently hydrolysing the latter to sulpho groups and, if desired, converting a resultant compound into the corresponding salt or converting a resultant salt into the corresponding free acid.

2. A process according to claim 1 for the preparation of a phthalocyanine compound of the formula (1), wherein q is 0, which process comprises carrying out the reaction of the sulphonamide in b) with $n_1$ molar equivalents of the halide of the formula $Hal–SO_2–R_3$.

3. A process according to claim 1 or 2 for the preparation of a phthalocyanine compound of the formula (1), wherein m is hydrogen, an alkali metal ion, an ammonium ion or an amine salt ion, $R_2$ is a halogen atom or cyano, X is hydrogen, ammonium, or an alkali metal atom, which process comprises reacting a compound of the formula (3), wherein $R_2$ is in each case as defined above and the remaining symbols are as defined in claim 1 and $n_1$ is any number between 2 an 4, according to reaction (a), b) or c), where $R_3$ in the halide $Hal–SO_2–R_3$ is as defined in claim 1.

4. A process according to claim 3, wherein $R_2$ is a halogen atom and $R_3$ is $C_1$–$C_4$alkyl, $C_1$–$C_4$haloalkyl, $C_2$–$C_6$alkoxyalkyl, $C_1$–$C_4$hydroxyalkyl, $C_2$–$C_5$cyanoalkyl, di–$C_1$–$C_4$alkylamino-$C_1$–$C_4$ alkyl or benzyl, phenyl or phenyl substituted by 1 to 3 substituents from the group halogen, $C_1$–$C_4$alkyl, $C_1$–$C_4$alkoxy, cyano, nitro, di–$C_1$–$C_4$alkylamino and sulpho and carboxyl and the salts thereof, which process comprises reacting a compound of the formula (3), wherein $R_2$ is in each case as defined above and the remaining symbols are as defined in claim 1 and $n_1$ is any number between 2 and 4, according to reaction a), b) or c), where $R_3$ in the halide $Hal–SO_2–R_3$ is as defined above.

5. A process according to claim 4 for the preparation of a phthalocyanine compound of the formula

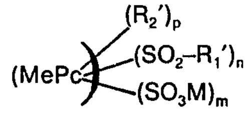

(2),

wherein $R_2'$ is chlorine, bromine or iodine, in particular chlorine, M' is hydrogen, sodium, potassium or ammonium, and R' is hydrogen, M' or a group of the formula

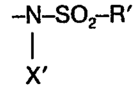 or 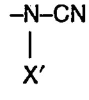

wherein X′ is hydrogen, sodium, potassium or ammonium, and $R_3'$ is $C_1$–$C_4$alkyl, phenyl, $C_1$–$C_4$alkylphenyl, chlorophenyl or methoxyphenyl, and MePc, n, m and p are as defined in claim 3, preferably those of the formula (2), wherein $R_2'$ is chlorine, M′ is hydrogen, sodium or potassium, X′ is hydrogen, sodium or potassium, $R_3'$ is $C_1$–$C_4$alkyl, phenyl or $C_1$–$C_4$alkylphenyl, p is a number from 0.5 to 1.5, n is a number from 2 to 4 and m is zero, which process comprises reacting 1 molar equivalent of a phthalocyanine sulphohalide of the formula

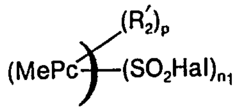

wherein $R_2'$ and p are as defined above, Hal is a halogen atom and $n_1$ is any number from 1 and [sic] 4, preferably from 2 to 4,

a) with $n_1'$, preferably $n_1$, molar equivalents of cyanamide or a salt thereof, or

b) with $n_1'$, preferably $n_1$, molar equivalents of ammonia, and reacting the resultant sulphonamide with $n_1$ molar equivalents of a halide of the formula Hal–$SO_2$–$R_2'$, wherein R′ is as defined above and Hal is a halogen atom, preferably a chlorine atom, or

c) with $n_1'$, preferably $n_1$, molar equivalents of a compound of the formula $H_2N$–Y, wherein Y is amino, unsubstituted or substituted alkylamino or dialkylamino, cyclohexylamino or N-acylamino, preferably with hydrazine, where n′ is a number $n_1$ and, in a product obtained according to a) to c) which still contains sulphohalide groups simultaneously or subsequently hydrolyzing the latter to sulpho groups.

6. A process according to claim 2 or 5 for the preparation of a pathalo-cyanine compound of the formula (1) or (2), wherein q is 0, and $R_1$ or $R_1'$ is hydrogen or a group of the formula

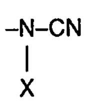 or 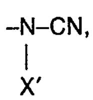

preferably a group of the formula

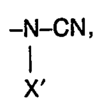

which process comprises reacting one molar equivalent of a sulphochloride of the formula

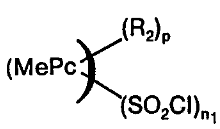 or 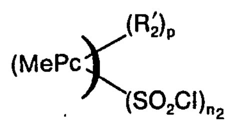

wherein $R_2$, $R_1'$, p and $n_1$ are as defined in claims 2 and 5 respectively,

a) with $n_1'$, preferably $n_1$, molar equivalents of cyanamide or a salt thereof, or

b) with $n_1'$, preferably $n_1$, molar equivalents of a compound of the formula $H_2$–N–Y, wherein Y is as defined in claim 5, preferably hydrazine, where $n_1'$ is a number $\leqslant n_1$, reaction a) being preferred, and, in a product obtained according to a) or b) which still contains sulphohalide groups simultaneously or subsequently hydrolysing the latter to sulpho groups.

7. A process for the preparation of a phthalocyanine compound of the formula (1) wherein $R_1$ is a group of the formula $-NXSO_2-R_3$, which process comprises reacting 1 molar equivalent of a phthalocyanine sulphohalide of the formula (3) with $n_1'$ molar equivalents of a sulphonamide of the formula $R_3$–$SO_3NH_2$, wherein $R_3$ is as defined in claim 1, where $n_1'$ is a number $\leqslant n_1$, and, in a product obtained which still contains sulphohalide groups simultaneously or subsequently hydrolyzing the latter to sulpho groups.

8. A process according to claim 1 for the preparation of a phthalocyanine compound of the formula

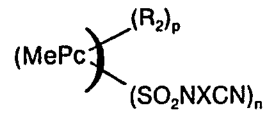

wherein the general symbols are as defined in claim 1, which process comprises reacting one molar equivalent of a sulphochloride of the formula

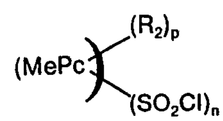

with about n molar equivalents of cyanamide and, if desired, converting the resultant compound, where X = H, into a salt.

**Revendications pour les Etats Contractants BE, CH, DE, FR, IT, LU, NL, SE, LI**

1. Composés phtalocyanines de formule

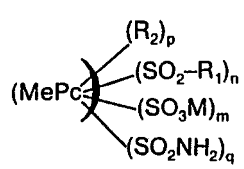

dans laquelle MePc désigne le système cyclique phtalocyanine de zinc ou d'aluminium, M désigne l'hydrogène ou l'équivalent d'un cation salifiable, et $R_1$ désigne M ou un groupe de formule

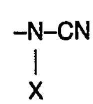 ou 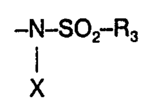

où X désigne l'hydrogène, l'ammonium ou l'équivalent d'un ion métallique mono-, bi- ou trivalent, $R_3$ désigne un radical alkyle en $C_1$–$C_8$, non-substi-

tué ou substitué par un radical hydroxy, alcoxy en $C_1$–$C_8$, halogéno, cyano, phényle, carb(alcoxy en $C_1$–$C_8$), aminocarbonyle ou di(alkyle en $C_1C_8$)-amino, un radical phényle non-substitué ou substitué par un radical alkyle en $C_1$–$C_8$, alcoxy en $C_1$–$C_8$, nitro, halogéno(alkyle en $C_1$–$C_8$), halogéno, (alcoxy en $C_1$–$C_8$)-carbonyle, cyano, alkylsulfonyle en $C_1$–$C_8$, carboxy et sulfo, ou leurs sels, esters ou amides, ou encore par un radical trifluorométhyle ou di-(alkyle en $C_1$–$C_8$)amino, le radical naphtyle, un noyau hétérocyclique aromatique, non-substitué, substitué et/ou condensé, à 5 ou 6 chaînons, comportant un ou deux atomes d'azote, d'oxygène ou de soufre, lequel noyau peut être substitué comme le groupe aromatique $R_3$, chaque radical $R_2$ est un radical halogéno, phényle ou cyano, n a une valeur de 1 à 4, m et q valent chacun de 0 à 3, et p a une valeur de 0 à 4, chacune des sommes n + m et n + q valant de 1 à 4, mais q n'étant pas égal à 0 quand $R_1$ est un groupe de formule

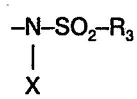

et m = 0

2. Composés phtalocyanines selon la revendication 1, dans lesquels q = 0.

3. Composés phtalocyanines selon la revendication 1, dans lesquels M est l'hydrogène ou un ion métal alcalin, ammonium ou sel d'amine, $R_2$ est un radical halogéno ou cyano, et X est un hydrogène, l'ammonium ou un métal alcalin.

4. Composés phtalocyanines selon la revendication 3, dans lesquels $R_2$ est un halogène et $R_3$ est un radical alkyle en $C_{1-4}$, halogéno-(alkyle en $C_{1-4}$), alcoxyalkyle en $C_{2-6}$, hydroxyalkyle en $C_{1-4}$, cyanoalkyle en $C_{2-5}$, di-(alkyle en $C_{1-4}$)amino-(alkyle en $C_{1-4}$), benzyle, phényle, phényle substitué par 1 à 3 radicaux halogéno, alkyle en $C_{1-4}$, alcoxy en $C_{1-4}$, cyano, nitro, di(alkyle en $C_{1-4}$)amino ou sulfo, ou encore un groupe carboxy, et leurs sels.

5. Composés phtalocyanines selon la revendication 4, caractérisés par la formule

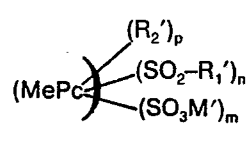

dans laquelle $R_2'$ est le chlore, le brome ou l'iode, M' est l'hydrogène, le sodium, le potassium ou l'ammonium, et $R_1'$ est M' ou un groupe de formule

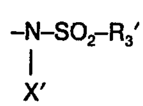 ou 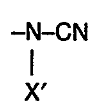

dans lesquelles X' est un hydrogène, le sodium, le potassium ou l'ammonium, $R_3'$ est un radical alkyle en $C_{1-4}$, phényle ou (alkyle en $C_{1-4}$)phényle, chlorophényle ou méthoxyphényle, et MePc, n, m et p sont comme définis dans la revendication 1.

6. Composés phtalocyanines selon la revendication 2, dans lesquels $R_1$ est un hydrogène ou un groupe de formule

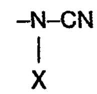

7. Composés phtalocyanines selon la revendication 5, dans lesquels $R_2'$ est le chlore

8. Composés phtalocyanines selon la revendication 5, dans lesquels $R_1'$ est un groupe de formule

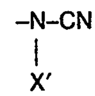

**Revendications pour l'Etat Contractant AT**

1. Procédé pour la préparation de composés phtalocyanines de formule

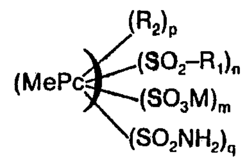 (1),

dans laquelle MePc désigne le système cyclique phtalocyanine de zinc ou d'aluminium, M désigne l'hydrogène ou l'équivalent d'un cation salifiable, et $R_1$ désigne M ou un groupe de formule

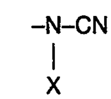 ou 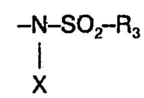

où X désigne l'hydrogène, l'ammonium ou l'équivalent d'un ion métallique mono-, bi- ou trivalent, $R_3$ désigne un radical alkyle en $C_1$–$C_8$, non-substitué ou substitué par un radical hydroxy, alcoxy en $C_1$–$C_8$, halogéno, cyano, phényle, carb(alcoxy en $C_1$–$C_8$), aminocarbonyle ou di(alkyle en $C_1$–$C_8$)-amino, un radical phényle non-substitué ou substitué par un radical alkyle en $C_1$–$C_8$, alcoxy en $C_1$–$C_8$, nitro, halogéno-(alkyle en $C_1$–$C_8$), halogéno, (alcoxy en $C_1$–$C_8$-carbonyle, cyano, alkylsulfonyle en $C_1$–$C_8$, carboxy et sulfo, ou leurs sels, esters ou amides, ou encore par un radical trifluorométhyle ou di(alkyle en $C_1$–$C_8$)amino, le radical naphtyle, un noyau hétérocyclique aromatique, non-substitué, substitué et/ou condensé, à 5 ou 6 chaînons, comportant un ou deux atomes d'azote, d'oxygène ou de soufre, lequel noyau peut être substitué comme le groupe aromatique $R_3$, chaque radical $R_2$ est un radical halogéno, phényle ou cyano, n a une valeur de 1 à 4, m et q valent chacun de 0 à 3, et p a une valeur de 0 à 4, chacune des sommes n + m est n + q valant de 1 à 4, mais q n'étant pas égal à 0 quand $R_1$ est un groupe de formule

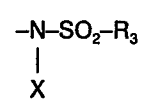

et m = 0,
caractérisé en ce qu'on fait réagir un équivalent molaire d'un sulfohalogénure de phtalocyanine de formule

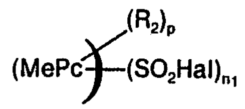

(3),

dans laquelle MePc, $R_2$ et p sont comme définis ci-dessus, Hal est un atome d'halogène, en particulier un atome de chlore, et $n_1$ est un nombre quelconque entre 1 et 4,

a) sur $n_1'$ équivalents molaires de cyanamide ou de l'un de ses sels, ou

b) sur $n_1'$ équivalents molaires d'ammoniac, et le sulfonamide obtenu sur $n_1$ ou $n_1''$ équivalents molaires d'un halogénure de formule

$$Hal{-}SO_2{-}R_3,$$

dans laquelle Hal et $R_3$ sont comme définis ci-dessus, ou

c) sur $n_1'$ équivalents molaires d'un composé de formule $H_2N{-}Y$, dans laquelle Y désigne le radical amino, un radical alkylamino ou dialkylamino éventuellement substitué, ou encore le radical cyclohexylamino ou N-acylamino, de préférence sur l'hydrazine,

$n_1'$ et $n_1''$ étant chacun un nombre $\leqslant n_1$, le nombre d'équivalents molaires d'ammoniac utilisé dans le cas b) étant $n_1$ quand la réaction a lieu avec $n_1''$ équivalents molaires de $Hal{-}SO_2{-}R_3$, et que, éventuellement, simultanément ou après-coup, dans les produits obtenus selon a) à c) et contenant encore des groupes sulfohalogénure, on hydrolyse ces derniers en groupes sulfo et on convertit les composés éventuellement obtenus en les sels correspondants, ou encore on libère des sels obtenus les formes acides correspondantes.

2. Procédé selon la revendication 1 pour la préparation de composés phtalocyanines de formule (1) dans laquelle $q = 0$, caractérisé en ce que, dans le cas b), on effectue la réaction du sulfonamide sur $n_1$ équivalents molaires de l'halogénure de formule $Hal{-}SO_2{-}R_3$.

3. Procédé selon la revendication 1 ou 2, pour la préparation de composés phtalocyanines de formule (1) dans laquelle M est un hydrogène, ou un ion métal alcalin, ammonium ou sel d'amine, $R_2$ est un atome d'halogène ou le radical cyano, X est un hydrogène, l'ammonium ou un atome de métal alcalin, caractérisé en ce qu'on fait réagir selon la réaction a), b) ou c) un composé de formule (3) dans laquelle chaque radical $R_2$ est comme défini ci-dessus, et les autres symboles sont définis comme dans la revendication 1, et $n_1$ est un nombre quelconque compris entre 2 et 4, $R_3$, dans l'halogénure $Hal{-}SO_2{-}R_3$, étant comme défini dans la revendication 1.

4. Procédé selon la revendication 3, dans lequel $R_2$ est un atome d'halogène et $R_3$ est un radical alkyle en $C_1$–$C_4$, halogènalkyle en $C_1$–$C_4$, alcoxyalkyle en $C_2$–$C_6$, hydroxyalkyle en $C_1$–$C_4$. cyanoalkyle en $C_2$–$C_5$, di(alkyle en $C_1$–$C_4$)amino-(alkyle en $C_1$–$C_4$) ou benzyle, phényle, ou phényle substitué par 1 à 3 substituants du groupe comprenant les halogènes, les radicaux alkyle en $C_1$–$C_4$, alcoxy en $C_1$–$C_4$, cyano, nitro, di(alkyle en $C_1$–$C_4$)amino, et sulfo et carboxy ou leurs sels, caractérisé en ce qu'on fait réagir selon les réactions a), b) ou c) un composé de formule (3) dans laquelle chaque radical $R_2$ est comme défini ci-dessus et les autres symboles sont définis comme dans la revendication 1, et $n_1$ est un nombre quelconque entre 2 et 4, $R_3$, dans l'halogénure $Hal{-}SO_2{-}R_3$, étant défini comme ci-dessus.

5. Procédé selon la revendication 4, pour la préparation de composés phtalocyanines de formule

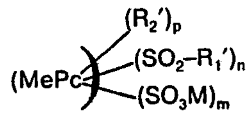

(2),

dans laquelle $R_2'$ est le chlore, le brome ou l'iode, en particulier le chlore, M' est un hydrogène, le sodium, le potassium ou l'ammonium, et $R_1'$ est un hydrogène, M' ou un groupe de formule

$$-\underset{X'}{\underset{|}{N}}-SO_2-R_3' \quad \text{ou} \quad -\underset{X'}{\underset{|}{N}}-CN$$

où X' est un hydrogène, le sodium, le potassium ou l'ammonium, et $R_3'$ est un radical alkyle en $C_1$–$C_4$, phényle, (alkyle en $C_1$–$C_4$)phényle, chlorophényle ou méthoxyphényle, et MePc, n, m et p sont comme définis dans la revendication 3, et de préférence de composés de formule (2) dans laquelle $R_2'$ est le chlore, M' un hydrogène, le sodium ou le potassium, X' un hydrogène, le sodium ou le potassium, $R_3'$ un radical alkyle en $C_1$–$C_4$, le radical phényle ou un radical (alkyle en $C_1$–$C_4$)-phényle, p un nombre de 0,5 à 1,5, n un nombre de 2 à 4 et m vaut 0, caractérisé en ce qu'on fait réagir un équivalent molaire d'un sulfohalogénure de phtalocyanine de formule

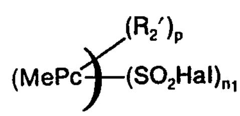

,

dans laquelle $R_2'$ et p sont comme définis ci-dessus, Hal est un atome d'halogène et $n_1$ un nombre quelconque de 1 à 4, de préférence de 2 à 4, sur

a) $n_1'$, de préférence $n_1$, équivalents molaires de cyanamide ou de l'un de ses sels, ou

b) $n_1'$, de préférence $n_1$, équivalents molaires d'ammoniac, et le sulfonamide obtenu sur $n_1$ équivalent molaire d'un halogénure de formule $Hal{-}SO_2{-}R_3'$, dans laquelle $R_3'$ est comme défini ci-dessus et Hal est un atome d'halogène, de préférence de chlore, ou

c) $n_1'$, de préférence $n_1$, équivalents molaires d'un composé de formule $H_2N{-}Y$, dans laquelle Y représente le radical amino, un radical alkylamino ou dialkylamino éventuellement substitué, ou encore le radical cyclo-hexylamino ou N-acylamino, de préférence sur l'hydrazine,

$n_1'$ étant un nombre $\leqslant n_1$, et éventuellement, simultanément ou après-coup, dans des produits

obtenus selon a) ou c) et contenant encore des groupes sulfo-halogénure, on hydrolyse ces derniers en groupes sulfo.

6. Procédé selon la revendication 2 ou la revendication 5, pour la préparation de composés phtalocyanines de formules respectivement (1) et (2), où q vaut 0 et les radicaux $R_1$ et $R_1'$ représentent chacun un hydrogène ou un groupe de formule

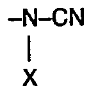 ou 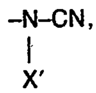

de préférence un groupe de formule

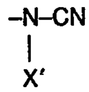

caractérisé en ce qu'on fait réagir un équivalent molaire d'un sulfochlorure de formule

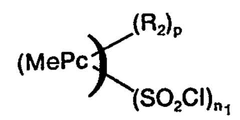 ou 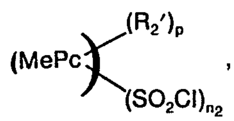 ,

où $R_2$, $R_2'$, p et $n_1$ sont commt définis dans les revendications 2 et 5,

a) sur $n_1'$, de préférence $n_1$, équivalents molaires de cyanamide ou de l'un de ses sels, ou

b) sur $n_1'$, de préférence $n_1$, équivalents molaires d'un composé de formule $H_2N-Y$, où Y est comme défini dans la revendication 5, de préférence sur l'hydrazine, $n_1'$ étant un nombre $\leqslant n_1$, la réaction a) étant préférée, et que, éventuellement, simultanément ou après-coup, dans les produits obtenus selon a) ou b) et contenant encore des groupes sulfohalogénure, on hydrolyse ces derniers en groupes sulfo.

7. Procédé pour la préparation de composés phtalocyanines de formule (1), dans laquelle $R_1$ est un groupe de formule $-NXSO_2-R_3$, caractérisé en ce qu'on fait réagir un équivalent molaire d'un sulfohalogénure de phtalocyanine de formule (3) sur $n_1'$ équivalents molaires d'un sulfonamide de formule $R_3-SO_3NH_2$, dans laquelle $R_3$ est comme défini dans la revendication 1, $n_1'$ étant un nombre $\leqslant n_1$, et que, éventuellement, simultanément ou après-coup, dans les produits obtenus contenant encore des groupes sulfohalogénure, on hydrolyse ces derniers en groupes sulfo.

8. Procédé selon la revendication 1 pour la préparation de composés phtalocyanines de formule

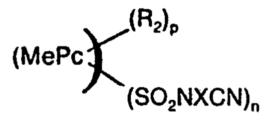 ,

dans laquelle les symboles généraux ont les significations données dans la revendication 1, caractérisé en ce qu'on fait réagir un équivalent molaire d'un sulfochlorure de formule

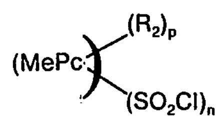

sur environ n équivalents molaires de cyanamide, et qu'on convertit éventuellement le composé obtenu, avec X = H, en un sel.